# EUROPEAN PATENT APPLICATION

(11) **EP 2 216 341 A1**
(43) Date of publication of application: **11.08.2010**
(21) Application number: 09001878.9
(22) Date of filing: 10.02.2009
(51) Int. Cl.: C07K 14/79

(54) **Transferrin variants and conjugates**

(71) Applicant: Novozymes Biopharma UK Limited, Nottingham, Nottinghamshire NG7 1FD (GB)
(72) Inventor: The designation of the inventor has not yet been filed
(74) Representative: Rasmussen, Preben

(57) **Abstract**

The application discloses variants of polypeptides of the transferrin family comprising at least on free thiol group on the surface, which polypeptide has reduced binding to at least one of the transferrin receptors. The polypeptides of the invention are useful for preparing conjugates with bioactive and/or therapeutic compounds, which compounds have high stability and long plasma halflife, which make these conjugates particular suitable for treatment of various diseases and disorders.

## Description

### Reference to sequence listing

This application contains a Sequence Listing in computer readable form. The computer readable form is incorporated herein by reference.

### FIELD OF THE INVENTION

The present invention relates to conjugates of a polypeptide and at least one bioactive compound, to polypeptides for making the conjugates, to polynucleotides encoding them and methods of making and using the recombinant protein. The polypeptide is recombinant transferrin mutant engineered with one of more reactive cysteine residues, and proteins comprising the sequence of these, particularly mutants that avoid glycosylation, which polypeptide has reduced or no binding to one or more transferrin receptors. The cysteine engineered recombinant transferrin mutants may be conjugated with chemotherapeutic drugs, toxins, polypeptides, and detection labels such as fluorophore and the conjugate will have reduced or no binding to one or more transferrin receptors.

### BACKGROUND OF THE INVENTION

It is known to use conjugates of transferrin with bioactive compounds for receptor-mediated endocytosis to improve the transcellular delivery. Thus, N.J. Kavimandam et al., Bioconjugate Chem., 2006, 17, 1376-1384 is titled "Synthesis and Characterization of Insulin-Transferrin Conjugates". D. Shah et al., Journal of Pharmaceutical Sciences, Vol. 85, No. 12, December 1996, is titled "Transcellular Delivery of an Insulin-Transferrin Conjugate in Enterocyte-like Caco-2 Cells" describe fusion proteins comprising a transferrin protein fused to a therapeutic protein. Fritzer et al., (1996) Biochem. Pharm., 51, 489-493 is titled "Cytotoxic Effects of a Doxorubicin-Transferrin Conjugate in Multidrug-Resistant KB Cells. US 20030221201 and 20040023334, and Friden, P. M, et al. WO9502421-A is titled "Methods for delivering a neuro-pharmaceutical agent across the blood brain barrier - which are non-invasive and allow transfer of the agent in an active form; Beug, H. et al. (1990) EP388758-A, EP104700 is called "Transferrin polycation conjugate - for transport of nucleic acids in cells via receptor mediated endocytosis, used e.g. to inhibit oncogenes in humans."

Faulk, W. WO8700756-A describes "Conjugate of transferrin with radioactive iodine - for use in tumour diagnosis, imaging, localisation or treatment.", WO8500812-A" Conjugates of transferrin or ceruloplasmin with anti-tumour agents - for treatment of tumours and determination of susceptibility of tumour cells to anti-tumour agents." describes targeting compounds into the tumor cells, designed for the diagnosis, imaging, localization.

Conventional means of attaching, a bioactive compound to a transferrin protein using conjugation generally leads to a heterogeneous mixture of molecules where the bioactive moieties are attached at a number of sites on the transferrin. Bioactive compounds such as cytotoxic drugs have typically been conjugated to transferrin through numerous (58) lysine residues, generating a heterogeneous transferrin -drug conjugate mixture. Visser, C. C., L. H. Voorwinden, et al. (2004). "Coupling of metal containing homing devices to liposomes via a maleimide linker: use of TCEP to stabilize thiol-groups without scavenging metals." J Drug Target 12(9-10): 569-73 describes methods for introduce a free thiol group(s) to transferrin using N-succinimidyl S-acetylthioacetate (SATA) at primary amine groups in order to couple transferrin to a liposome. Using this methodology introduction of thiol groups was 2.2 mol - SH per mol Tf in the presence of EDTA; and 5.6 mol -SH per mol Tf in the presence of TCEP. A given particular integer ratio of bioactive moieties to transferrin, is also potentially a heterogeneous mixture where the drug moiety is attached at various sites on the transferrin. In addition, the multistep conjugation process may be difficult to control and therefore nonreproducible. Cysteine thiols are reactive at neutral pH, introduction of one or more thiol reactive groups to transferrin would allow site-directed specific coupling of a bioactive compound.

Human serum transferrin (HST) is known to be a single-chain polypeptide of 679 amino acid residues which contain 38 cysteine residues linked in 19 disulfide bridges with the capacity to bind two ferric ions. The three-dimensional structure of human transferrin was published in J. Wally et al., Journal of Biological Chemistry, 281 (34), 24934-24944 (2006). According to the human transferrin crystal structure from Wally *et al.,* HST comprises an N-lobe/domain consisting of amino acids 1-331, a C-lobe/domain consisting of amino acids 339-679, and an interlobe linker consisting of amino acids 332-338. Each of the two transferrin lobes is able to reversibly bind a single ferric ion (Fe³⁺) (Aisen *et al*., 1978;Aisen, 1998;Evans and Williams, 1978;Lambert *et al.,* 2005) and a synergistic carbonate anion is also bound (Baker, 1994).

When a transferrin protein loaded with iron encounters a transferrin receptor (TfR) on the surface of a cell, it binds to it and is consequently transported into the cell in a vesicle. The cell will acidify the vesicle, causing transferrin to release its iron ions. The receptor is then transported through the endocytic cycle back to the cell surface, ready for another round of iron uptake.

Cheng, Y., Cell (Cambridge, Mass.) v116, pp. 565-576 (2004) describes a model for the structure of the Human Transferrin Receptor-Transferrin Complex.

The structure is found as 1SUV in the Protein Data Bank and describes binding of human serum transferrin to human transferrin receptor 1.

J. Wally et al., Journal of Biological Chemistry, 281 (34), 24934-24944 (2006) describes a three-dimensional structure of iron-free human transferrin. The structure is found as 2HAV in the Protein Data Bank.

The transferrins form a group of proteins with high sequence homology, including human serum transferrin (HST), lactoferrin, melanotransferrin and ovotransferrin.

Native HST is known to contain two lobes (N- and C-lobes) with 19 disulfide bridges, an O-glycosylation site at serine-32 (S32), and two N-glycosylation sites at asparagine 413 (N413) and asparagine 611 (N611).

Woodworth, R. C., et al. (1991), Biochemistry 30, 10824-9, discloses mutants of the N-terminal half-molecule of human serum transferrin including the mutant D63C. Muralidhara, B. K. and Hirose, M. (2000), Protein Sci 9, 1567-1575, discloses selective reduction of the isolated C-lobe of ovotransferrin. J. Williams et al., Biochem. J. (1985) 228, 661-665 discloses selective reduction of a disulphide bridge in ovotransferrin or the C-terminal half-molecule. US 5986067 (Funk et al.) discloses a recombinant HST mutant which does not allow glycosylation.

More than one type of receptor for transferrin family proteins is known. The receptors for human serum transferrin include transferrin receptor 1, transferrin receptor 2 and cubulin, receptors for human lactoferrin are also known. Members of the transferrin family of proteins have different binding properties to different transferrin receptors.

Three receptors are known for human serum transferrin. Transferrin Receptor 1 (p90, CD71, TfR1, TFRC), transferrin receptor 2 (TfR2) and cubulin.

Receptors have also been identified for other transferrin family members; several receptors have been identified for lactoferrin reviewed in Suzuki, Y.A., Lopez, V., and Lonnerdal, B. 2005. Mammalian lactoferrin receptors: structure and function. Cellular and Molecular Life Sciences 62:2560-2575.

Melanotransferrin (p97) has high protein sequence similarity with transferrin and lactoferrin however, no receptor has been identified. Melanotransferrin predominantly occurs as a membrane bound, glycosylphosphatidylinositol (GPI) anchored molecule (Rose, T.M, et al. (1986) Primary structure of the human melanoma-associated antigen p97 (melanotransferrin) deduced from the mRNA sequence. Proc. Natl. Acad. Sci. U. S. A 83:1261-1265, but a soluble form produced by alternative splicing also exists Food, M.R., et al (2002). The soluble form of the membrane-bound transferrin homologue, melanotransferrin, inefficiently donates iron to cells via nonspecific internalization and degradation of the protein. Eur. J. Biochem. 269:4435-4445. WO/2002/013873, WO2003009815-A; and WO2003009815-A2 describes melanotransferrin conjugates and their methods of use

Different animal transferrins may have different receptor binding capability, for example Kawabata et al. (2004) Br. J. Haemato/. 127:464-473 demonstrate that bovine and human serum transferrins interact with TfR2; human serum transferrin interacts with TfR1 but bovine transferrin does not; human melanotransferrin is not able to bind either TfR1 or TfR2.

US2003/026778 (Prior, Christopher, P *at al*) discloses "Oral Delivery Of Modified Transferrin Fusion Proteins and US2003/026818 (Prior, Christopher, P et al.) claims "Modified Transferrin Fusion Proteins", and the University of Southern California/US government PCT patent WO/2005/034877 claims G-CSF-Transferrin fusion protein, for example, conjugate and recombinant proteins, for oral or subcutaneous administration. US7176278 claims transferrin fusion proteins in which the transferrin moiety may be modified to reduce or inhibit glycosylation, or modified so that it is unable to bind to a transferrin receptor, to iron or to bicarbonate.

### SUMMARY OF THE INVENTION

In the co-pending patent application PCT/EP2008/060482 the inventors disclose transferrin variants having one or more free cysteine residues on the surface of the protein and which have normal receptor binding.

In a first aspect the present invention relates to transferrin variants having one or more free cysteine residues on the surface of the protein and which transferrin variants when conjugated to a bioactive molecues have reduced binding to at least one transferrin receptor.

In a second aspect the invention relates to a conjugate comprising the transferrin variant according to the invention and a therapeutic moiety bound to the transferrin variant via one or more cysteine residues free thiol groups on the surface and which conjugate has reduced binding to at least one of the transferrin receptors.

The inventors have realized that transferrin variants having reduced binding to at least one transferrin receptors have the same desirable properties as unmodified transferrin, such as high stability in plasma high in vivo plasma half-life and can be assimilated from the gastrointestinal tract, but because of the reduced binding to at least one transferrin receptor the transferrin variants according to the invention will in vivo be localised to the plasma to a higher extent than normal transferrin and in a less degree be bound to transferrin receptors. Conjugates comprising the transferrin variant according the invention and at least one therapeutic moiety bound to the transferrin variant via the at least one free cysteine residues on the surface of the transferrin variant will also have similar beneficial properties as the unmodified transferrin but will remain in the blood stream and not to a large extend be localized the fast growing cells having a high density of transferrin receptors. Thus, the transferrin variants according to the invention have a different compartmentalization in the human body and tend to be localized in the circulation.

Accordingly, the invention provides a polypeptide conjugated to one or more bioactive moieties which may or may not have iron binding capacity and have a binding capacity of less than 5% to one or more transferrin receptor

The polypeptide has an amino acid sequence which is at least 40 % or at least 60 % identical to residues 1-679 or, 1-331, 339-679 of SEQ ID NO: 1, residues 1-691 of SEQ ID NO:2 or residues 1-738 of SEQ ID NO:3 and comprises one or more cysteine residues with a free thiol group.

Based on a three-dimensional structure of a transferrin, the inventors have designed variant polypeptides (muteins) which have one or more cysteine residues with a free thiol group (hereinafter referred to as "thiotransferrin") which also have no or reduced binding to one or more transferrin receptor transferrin receptor. The variant polypeptide may be conjugated through the sulphur atom of the cysteine residue of the polypeptide to a bioactive compound.

The term thiotransferrin is used herein to describe a transferrin variant which comprises one or more unpaired cysteines, which when conjugated with a bioactive molecule has a binding capacity of less than 5% to one or more transferrin receptor. Similar the terms thiolactoferrin and thiomelanotransferrin are used to describe variants of lactoferrin and melanotransferin respectively, which comprises one or more unpaired cysteine residues with free thiol groups.

The free thiol group of thiotransferrin may be created by insertion of a cysteine residue (the amino acid chain length is increased), substitution of two or more adjacent residues with a cysteine (the amino acid chain length is decreased) or substitution of an amino acid residue with a cysteine (the amino acid chain length is unchanged), deletion of a disulfide cysteine or combinations of the above. Site specific conjugation allows preparation of highly homogenous thiotransferrin conjugates, superior to transferrin conjugates prepared by non-site specific conjugation chemistry.

The invention describes a conjugated thiotransferrin protein which has a binding capacity of less than 5% to one or more transferrin receptor compared to a wild- type transferrin sequence. In one embodiment the thiotransferrin may contain modifications to the Transferrin receptor binding site(s). In its unconjugated state thiotransferrin molecule may have a binding capacity of more than 5% to one or more transferrin receptor, however when the thiotransferrin molecule is conjugated with a bioactive compound it will have a binding capacity of less than 5% to one or more transferrin receptor, compared with the unmodified transferrin.

In another embodiment, the unconjugated thiotransferrin molecule has binding capacity of less than 5% to one or more transferrin receptor, and when the thiotransferrin molecule is conjugated with a bioactive compound it will have a binding capacity of less than 5% to one or more transferrin receptor, compared with the unmodified transferrin.

In another embodiment the thiotransferrin mutein may also be modified to exhibit reduced or no binding to iron or carbonate. Iron loaded diferric human transferrin binds with higher affinity than monoferric human transferrin or human iron free transferrin to human transferrin receptor 1, human serum transferrin muteins with reduced or no binding to iron would be expected to have reduced or no binding to human transferrin receptor 1. In human serum transferrin, the iron binding sites comprise at least amino acids Asp 63, Asp 392, Tyr 95, Tyr 426, Tyr 188, Tyr 517, His 249, and His 585; the carbonate binding sites comprise at least amino acids Thr 120, Thr 452, Arg 124 and Arg 456 (Lambert, Perri, and Meehan, 2005).

Thiotransferrin may be made with any transferrin protein, fragment, or lobe/domain, or engineered lobe/domain or a transferrin fusion protein.

The thiotransferrin mutein may also have reduced or no glycosylation. The sequence of HST contains three glycosylation sites, two N-linked glycosylation sites (N413 and N611 in HST) and one O-linked glycosylation site (S32 in HST). Thus the thiotransferrin mutein described may also be modified in such that it is mutated to an amino acid which does not allow glycosylation at the two N-linked glycosylation sites or one O-linked glycosylation site or combinations of the above.

Preferably, the modified thiotransferrin variants comprise a human transferrin moiety engineered with one of more reactive cysteine residues and modified to reduce or prevent glycosylation; when the thiotransferrin is conjugated to a bioactive compound the conjugate has a binding capacity of less than 5% to one or more transferrin receptor.

In another aspect the invention relates to conjugates comprising at least one bioactive compound and a polypeptide of the invention.

### DETAILED DESCRIPTION OF THE INVENTION

### Transferrin

Transferrins are according to this invention intended to be understood as human serum transferrin (HST) and related proteins belonging to same family which in its natural form is capable of binding iron and further binds to the at least one transferrin family receptor. Detailed teaching on transferrin and proteins belonging to the transferrin family can be found in the co-pending patent application PCT/EP2008/060482 which is included in this application by reference, and the disclosure in this application in respect of transferrin and proteins in the transferrin family also apply for this application.

For clarity the thiotransferrin decribed in the invention may be any protein which belongs to the transferrin family as described, e.g., by Lambert et al., Comparative Biochemistry and Physiology, Part B, 142 (2005), 129-141, and by Testa, Proteins of iron metabolism, CRC Press, 2002; Harris & Aisen, Iron carriers and iron proteins, Vol. 5, Physical Bioinorganic Chemistry, VCH, 1991.

Examples of transferrin family proteins are serum transferrin, ovotransferrin, melanotransferrin and lactoferrin and their derivatives and variants, such as mutant transferrins (Mason et al., (1993) Biochemistry, 32, 5472; Mason et al., (1998), Biochem. J., 330, 35), truncated transferrins, transferrin lobes (Mason et al., (1996) Protein Expr. Purif., 8, 119; Mason et al., (1991) Protein Expr. Purif., 2, 214), mutant lactoferrins, truncated lactoferrins, lactoferrin lobes, mutant ovotransferrin, truncated ovotransferrin, melanotransferrin lobes, truncated melanotransferrin or fusions of any of the above to other peptides, polypeptides or proteins (Shin et al., (1995) Proc. Natl. Acad. Sci. USA, 92, 2820; Ali et al., (1999) J. Biol. Chem., 274, 24066; Mason et al., (2002) Biochemistry, 41, 9448). Serum transferrins are preferred, particularly a human serum transferrin (HST) having the amino acid sequence of SEQ ID NO: 1 with 679 amino acids, also called the C1 variant (Accession number NP_001054). Lactoferrins are preferred, particularly a human lactoferrin having the amino acid sequence of SEQ ID NO: 2 with 691 amino acids. Melanotransferrins are preferred, particularly human melanotransferrin having the amino acid sequence residue 20-738 of SEQ ID NO: 3.

The transferrin generally has an amino acid sequence which has at least 25% identity to SEQ ID NO: 1, particularly at least 30%, 35%, 40%, 45%, 50%, 55%, 60%, 65%, 70%, 75%, 80%, 85%, 90%, 91%, 92%, 93%, 94%, 95%, 96%, 97%, 98%, 99%, 99.1%, 99.2%, 99.3%, 99.4%, 99.5%, 99.6%, 99.7% or 99.8%The lactoferrin generally has an amino acid sequence which has at least 25% identity to SEQ ID NO: 2, particularly at least 30%, 35%, 40%, 45%, 50%, 55%, 60%, 65%, 70%, 75%, 80%, 85%, 90%, 91%, 92%, 93%, 94%, 95%, 96%, 97%, 98%, 99%, 99.1%, 99.2%, 99.3%, 99.4%, 99.5%, 99.6%, 99.7% or 99.8%.

The melanotransferrin generally has an amino acid sequence which has at least 25% identity to SEQ ID NO: 3, particularly at least 30%. 35%, 40%, 45%, 50%, 55%, 60%, 65%, 70%, 75%, 80%, 85%, 90%, 91%, 92%, 93%, 94%, 95%, 96%, 97%, 98%, 99%, 99.1%, 99.2%, 99.3%, 99.4%, 99.5%, 99.6%, 99.7% or 99.8%.

The amino acid sequence of the transferrin family protein may have a length of at least 167 amino acids, particularly at least 300 amino acids, 630 amino acids, 691 amino acids, 694 amino acids or 695 amino acids. The length is typically at most 1274 amino acids, particularly at most 819 amino acids, at most 722 amino acids, at most 717 amino acids or at most 706 amino acids. The length may particularly be 679 amino acids. It may be a transferrin from vertebrae with 691-717 amino acids or a mammalian transferrin with a length of 695-706 amino acids.

The transferrin family protein, particularly one having a length of 694-706 amino acids, generally contains two lobe/domains (called the N- and C-lobes), each having around 331-341 residues and each binding one atom of Fe (III) and one carbonate anion, connected by an interlobe linker of about 7 residues. Typically, each iron is coordinated to four conserved amino acid residues: one Asp, two Tyr and one His, and the anion is bound to an Arg and a Thr. The thiotransferrin may be a mutein of full-length transferrin sequence, it may comprise a single transferrin lobe/domain, such as an individual N or C lobe/domain, or it may even comprise two or more lobes/domains, such as two N lobes or two C lobes.

In some embodiments, the use of a single N lobe/domain of the transferrin molecule may be advantageous since the majority of the TfR1 binding activity is assumed to be in the C lobe/domain, and the N lobe/domain, on its own, does not bind TfR1, alternatively the thiotransferrin may comprise two or more N terminal transferrin lobe/domains. The primary receptor-recognition site of HST for the TfR is in the C-lobe, and proteolytically isolated HST C-lobe is able to deliver ferric iron to cells.

In some embodiments the C-terminal lobe/domain of thiotransferrin is modified to function as an N-like lobe/domain with iron binding properties substantially like that of a native or wild-type N lobe/domain or lobe. As the transferrin receptor binding sites are located within the C-lobe then such thiotransferrins will have reduced binding to TfR1.

The transferrin may optionally be fused to another protein, particularly a bioactive protein such as those described below. The fusion may be at the N- or C-terminal or comprise insertions. The skilled person will also appreciate that the open reading frame may encode a protein comprising any sequence, be it a natural protein (including a zymogen), or a variant, or a fragment (which may, for example, be a lobe/domain) of a natural protein; or a totally synthetic protein; or a single or multiple fusion of different proteins (natural or synthetic). Examples of transferrin fusions are given in US patent applications US2003/026778, US2003/0221201 and US2003/0226155, Shin, et al., 1995, Proc Natl Acad Sci U S A, 92, 2820, Ali, et al., 1999, J Biol Chem, 274, 24066, Mason, et al., 2002, Biochemistry, 41, 9448, the contents of which are incorporated herein by reference.

### 3D model

The 3D model used in the invention comprises at least one molecule of transferrin and at least one receptor.

Other models including a transferrin family protein and a receptor can be built similarly on the basis of published 3D structures such as 1CB6, 1B0L, 1LFG, 1DTZ, 1AIV, 1DOT, 1OVT, 1H76, 1JNF, 2HAV and 1 SUV (Protein Data Bank).

### Model/Method 1 Selection of amino acid residues and regions on transferrin suitable for modification to introduce a free thiol group, which when conjugated with a bioactive molecule reduce binding to transferrin receptor 1- Step 1

Based on the location of the C-alpha atom of each residue in the 3D model, residues are selected which meet the criteria below. The model used was the exact same as in PCT/EP2008/060482, however, the accessibility calculations are new: Model A and B, were saved individually to PDB files, and analysed independently using DSSP (same method as in PCT/EP2008/060482), which gave the coordinates and distances disclosed in table 1.
1) The receptor binding area of transferrin was identified by selecting all amoni acid residues in chains A and Bm which have at least one heavy atom within a cutoff distance (6, 8 or 10 Angstroms) from any heavy atom in chain R or S.
2) The atom coordinates of chain A (transferrin model A) were saved to a separate PDB file. Likewise, the atom coordinates of chain B (transferrin model B) were saved to another PDB file. The analyses described in step 3) and 4) were performed independently of these PDB files.
3) For each of the transferrin models A and B, the solvent accessible surface area was calculated for each residue in the transferrin A and B models, using the DSSP software (W. Kabsch and C. Sander, Biopolymers 22 (1983) 2577-2637). Each solvent accessible surface area was divided by a standard value for the particular amino acid found in that position and multiplied by 100, thereby obtaining a percentage of the standard value for each residue.
The standard solvent accessible surface area for the 20 different amino acids are defined as (using one-letter codes for the amino acids):
A=62, C=92, D=69, E=156, F=123, G=50, H=130, I=84, K=174, L=97, M=103,
N=85, P=67, Q=127, R=211, S=64, T=80, V=81, W=126 and Y=104.
4) For each of the transferrin models A and B, the secondary structure was determined for each residue in the transferrin A and B models using the DSSP software. If the secondary structure is defined as H (Helix), B (isolated beta bridge) or E (Extended sheet), the residue is marked '1', otherwise as '0'.

**Table 1 shows data for residues in the two Tf chains in a 3D model (method 1).**

| Residue | Amino acid | Distance (A) ≤ | Distance (B) ≤ | % SASA (A) ≥ | % SASA (B) ≥ |
|---|---|---|---|---|---|
| 2 | T | | 8 | | 90 |
| 44 | D | | 8 | | 90 |
| 50 | A | | 6 | | 100 |
| 51 | A | | 6 | | 120 |
| 52 | N | | 6 | | 90 |
| 66 | D | 6 | | 100 | |
| 69 | L | | 6 | | 120 |
| 70 | A | 6 | 6 | 140 | 130 |
| 71 | P | 6 | 6 | 140 | 140 |
| 137 | P | | 8 | | 120 |
| 138 | E | | 6 | | 110 |
| 139 | P | 6 | 6 | 120 | 140 |
| 141 | K | 6 | | 90 | |
| 142 | P | | 8 | | 110 |
| 252 | S | 6 | | 90 | |
| 253 | M | 6 | 8 | 140 | 140 |
| 254 | G | 8 | 10 | 120 | 140 |
| 304 | P | 10 | | 140 | |
| 306 | M | 8 | | 90 | |
| 307 | D | 6 | | 90 | |
| 314 | Y | 10 | | 100 | |
| 323 | L | 6 | 6 | 120 | 110 |
| 324 | R | 6 | | 110 | |
| 326 | G | | 6 | | 140 |
| 327 | T | 10 | 6 | 130 | 120 |
| 329 | P | | 10 | | 140 |
| 330 | E | 6 | | 90 | |
| 331 | A | | 10 | | 140 |
| 332 | P | 8 | 10 | 100 | 110 |
| 333 | T | 8 | 8 | 140 | 140 |
| 334 | D | | 10 | | 140 |
| 344 | L | 6 | | 90 | |
| 346 | H | 6 | | 90 | |
| 347 | H | 6 | | 100 | |
| 357 | V | 6 | 8 | 140 | 130 |
| 358 | N | 8 | | 120 | |
| 360 | V | 6 | 6 | 140 | 140 |
| 369 | E | 6 | | 100 | |
| 499 | G | 10 | | 140 | |
| 500 | L | 6 | | 120 | |
| 507 | N | 8 | | 140 | |
| 611 | D | 10 | | 130 | |
| 613 | 5 | 6 | | 140 | |
| 614 | G | 6 | | 130 | |
| 623 | T | 6 | | 140 | |

Based on the data provided in table 1 residues suitable to be modified in order to provide transferrin variants having reduced receptor binding when conjugated to a bioactive moiety were selected based on the criteria below.
- Distance to the transferrin receptor <10 Angstroms, <8 Angstroms, <6 Angstroms
- And accessibility >90%,

The following lists amino acid residues selected by this criteria. The numbering of residues disregards the first three residues in SEQ ID NO: 1 which are unresolved in the 3D model and thus starts with K4 of SEQ ID NO: 1 as position 1.
**≤ 10 Angstroms, Accessibility ≥90 %**
   T 2 + D 44 + A 50 + A 51 + N 52 + D 66 + L 69 + A 70 + P 71 + P 137 + E 138 + P 139 + K 141 + P 142 + S 252 + M 253 + G 254 + P 304 + M 306 + D 307 + Y 314 + L 323 + R 324 + G 326 + T 327 + P 329 + E 330 + A 331 + P 332 + T 333 + D 334 + L 344 + H 346 + H 347 + V 357 + N 358 + V 360 + E 369 + G 499 + L 500 + N 507 + D 611 + S 613 + G 614 + T 623
**≤ 8 Angstroms, Accessibility ≥90 %**
   T 2 + D 44 + A 50 + A 51 + N 52 + P 66 + L 69 + A 70 + P 71 +P 137 + E 138 + P 139 + K 141 + P 142 + S 252 + M 253 + G 254 + M 306 + D 307 + L 323 + R 324 + G 326 + T 327 + E 330 + P 332 + T 333 + L 344 + H 346 + H 347 + V 357 + N 358 + V 360 + E 369 + L 500+ N 507 + S 613 + G 614 + T 623
**≤ 6 Angstroms, Accessibility ≥90 %**
   A 50 + A 51 + N 52 + D 66 + L 69 + A 70 + P 71 + E 138 + P 139 + K 141 + S 252 + M 253 + D 307 + L 323 + R 324 + G 326 + T 327 + E 330 + L 344 + H 346 + H 347 + V 357 + V 360 + E 369 + L 500 + S 613 + G 614 + T 623

Most preferred residues are those selected by both model A and model B which are ≤6A from the receptor with accessibility ≥90 %
**≤6 Angstroms, Accessibility ≥90 %**
   A70+P71 +P139+L323+V360

### Model/Method 1 Selection of amino acid residues and regions on transferrin suitable for modification to introduce a free thiol group, which when conjugated with a bioactive molecule reduce binding to transferrin receptor 1- Step 2

The first three N terminal amino acids, V 1, P 2 and D 3 are unresolved (Wally et al., (2006) Journal of Biological Chemistry, 281 (34), 24934-24944), so the assigned position numbers of the selected amino acid shown above are corrected for the addition of the unresolved three N terminal amino acids

Further, residues and regions may be selected on the basis of their relation to secondary structures in the 3D model (alpha-helices and beta-strands). An analysis on the basis of Table 2 of J. Wally et al., Journal of Biological Chemistry, 281 (34), 24934-24944 (2006) or table 1 of this application, indicates that the selected amino acids are located as follows in relation to secondary structures:

**Table 2**

| N-lobe | | Interlobe + linker C-lobe | |
|---|---|---|---|
| Amino acids | Structural location | Amino acids | Structural location |
| | | P332,E333, | Within interlobe linker |
| | | A334, P335, | |
| | | T336, D337, | |
| T5 | Beginning of βa | | |
| | | L347 | Loop between βa - α1 |
| | | H349 | Beginning of α1 |
| | | H350, V360 | Within α1 |
| | | N361 | End of α1 |
| | | V363 | Loop between α1-βb |
| | | E372 | Loop between βb - α2 |
| D47 | Within α2 | | |
| A53 | End of α2 | | |
| A54, N55 | Loop between α2-βc | | |
| D69 | α3 | | |
| L 72, A73, P74 | Loop between α3-βd | | |
| P140, E141, P142,K144, P145 | Loop between α5a - α6 | | |
| | | G502 | Beginning of α6a |
| | | L503 | Within α6a |
| S255,M256, G257 | Loop between βja - α9 | | |
| | | D614, S616, G617, T626, | Loop between α9 - βk |
| P307, M309, D310 | Loop between βk - α10 | | |
| Y317 | Beginning of α11 | | |
| L326 | Within α11 | | |
| R327 | End of α11 | | |
| G329, T330 | Loop between α11 and linker | | |

Based on the HST 3D structure described in the examples, the receptor distance, accessibility analysis performed and the mapping analysis performed above, the preferred regions for introduction of the one or more cysteine residues with a free thiol groups may be taken as all the loops, sheets and helices identified in the Table above, i.e. T5-A10, Y45-A53, A54-A59, A64-Y71, L72-N76, L139-P145, S255-K259, V305-D310, Y317-E328, G329- P341, G329-P341, L347-S348, H349-S362, V363-K365, A371-T373, G502-N504, G609-C637.

Most especially preferred positions for introduction of the one or more cysteine residues with a free thiol groups is defined as the amino acids identified in Table , which in either model A or B have a receptor distance ≤10 Angstroms, Accessibility ≥90 % i.e. T5+D47+A53+A 54 + N 55 + D 69 + L 72 + A 73 + P 74 + P 140 + E 141 + P 142 + K 144 + P 145 + S 255 + M 256 + G 257 + P 307 + M 309 + D 310 + Y 317 + L 326 + R 327 + G 329 + T 330 + P 332 + E 333 + A 334 + P 335 + T 336 + D 337 + L 347 + H 349 + H 350 + V 360 + N 361 + V 363 + E 372 + G 502 + L 503 + N 510 + D 614 + S 616 + G 617 + T 626

More preferred residues are those which have a receptor distance ≤8 Angstroms, Accessibility ≥90 % i.e. T 5 + D 47 + A 53 + A 54 + N 55 + D 69 + L 72 + A 73 + P 74 + P140 + E 141 + P 142 + K 144 + P 145 + S 255 + M 256 + G 257 + M 309 + D 310 + L 326 + R 327 + G 329 + T 330 + E 333 + P 335 + T 336 + L 347 + H 349 + H 350 + V 360 + N 361 + V 363 + E 372 + L 503 + N 510 + S 616 + G 617 + T 626

Even more preferred residues are those which have a receptor distance ≤6 Angstroms, Accessibility ≥90 % i.e A 53 + A 54 + N 55 + D 69 + L 72 + A 73 + P 74 + E 141 + P 142+ K 144 + S 255 + M 256 + D 310 + L 326 + R 327 + G 329 + T 330 + E 333 + L 347 + H 349 + H 350 + V 360 + E 372 + L 503 + S 616 + G 617 + T 626

Most preferred residues are those selected by both model A and model B which are ≤6 Angstroms from the receptor with accessibility ≥90 % i.e. A 73 + P 74+ P 142 + L 326 + V 363

### Alteration of transferrin amino acid sequence

A free thiol group may be introduced into the transferrin molecule by altering the amino acid sequence by substitution or insertion at a position selected as described above. Thus, the selected residue may be substituted with cysteine (the amino acid length is unchanged), or cysteine may be inserted at the N- or C-terminal side of the selected residue (the amino chain length is increased), or one or more adjacent residues including the selected residue may be substituted with cysteine (the amino acid chain length is reduced). Multiple alterations may be made to the polypeptide.

Method 1 describes a method for selection of amino acid residues and regions on transferrin suitable for modification to introduce a free thiol group which when conjugated with a bioactive molecule reduces binding to transferrin receptor 1. Method 1 is in particular with reference to transferrin, however, the skilled person will appreciate that the teaching applies likewise to other members of the transferrin family, such as lactoferrin and melanotransferrin.

There is more than one type of transferrin receptor known. Members of the transferrin family of proteins have different binding properties to different transferrin receptors. Three receptors are known for human serum transferrin, Transferrin Receptor 1 (p90, CD71 TFRC), transferrin receptor 2 (TfR2) and cubulin.

Transferrin Receptor 1 (p90, CD71 TFRC) is ubiquitously expressed on normal cells and at a higher amount on cells with a high proliferation rate. The elevated expresion of transferrin receptor 1 in cancer cells has been extensively reported (Daniels TR, et al. (2006). The transferrin receptor part I: Biology and targeting with cytotoxic antibodies for the treatment of cancer 3. Clin Immunol, 121, 144-158). Expression of TfR1 is regulated by iron levels.

In 1999 Kawabata *et al* cloned a second TfR-like molecule known as the transferrin receptor 2 (TfR2) (H Kawabata et al Molecular cloning of transferrin receptor 2 as a new member of the transferrin receptor-like family J. Biol. Chem 275 (1999) p20826-20832). Two transcripts are produced by alternative splicing, TfR2α and TfR2-β. TfR2-β transcript gives rise to an intracellular protein with unknown function. TfR2 is structurally similarity with TfR1, they exhibit a 45% similarity and 66% homology in their ectodomain but no similarity is observed between the cytoplasmic domains (Daniels TR, et al. (2006). The transferrin receptor part I: Biology and targeting with cytotoxic antibodies for the treatment of cancer 3. Clin Immunol, 121, 144-158). TfR2 and TfR1 differ in the cell surface expression and gene regulation and affinity for human serum transferrin. TfR2 has 25% lower affinity for transferrin than TfR1. TfR1 is expressed on all cells, except for mature erythrocytes and terminally differentiated cells, while human TfR2 is primarliy expressed in the liver and enterocytes in the small intestine and also on certain tumour cells. Unlike TfR1, TfR2 is not regulated by intracellular iron levels and appears to be regulated in relation with the cell cycle.

In addition a third receptor is known for human serum transferrin, Cubulin (intrinsic factor-cobalamin receptor; IFCR) which is expressed in polarised epithelia. Cubulin is a multi-ligand receptor. Megalin is a coreceptor for cubulin required for, cubilin-mediated endocytosis of Transferrin.

Cubulin is structurally distinct from present known human serum transferrin receptors.

Receptors have also been identified for other transferrin family members; several recetors have been identified for lactoferrin (reviewed in Suzuki, Y.A., etal (2005) Mammalian lactoferrin receptors: structure and function. Cellular and Molecular Life Sciences 62:2560-2575. At least three receptor have been described for lactoferrin (Campbell (1982) Suzuki *et al.* (2001) and Nagai, M.; *et al.* (2003). Suzuki *et al.* (2001) purified and cloned Intelectin 1 (ITLN1, lactoferrin receptor; LFR HL1) which they termed lactoferrin receptor (LfR), from human fetal intestine. RT-PCR detected high expression in fetal small intestine and adult heart and lower expression in Caco2 colon carcinoma cells. The structure of the receptor is structurally different from other TfR family proteins. Other tissue specfic LTfR have been described including Liver LfR2 (LV-LfR2, asisolglycoprotein receptor), Lymphocyte LfR (LC-LfR), monocyte macrophage LfR (MC LfR), platelet LfR (PL-LfR), mammary epithelial cell LfR (ME-LfR), respiratory epithelial cell LfR (RE-LfR), brain LfR (BR-LfR).

Unlike Transferrin and Lactoferrin, Melanotransferrin predominantly occurs as a membrane bound, glycosylphosphatidylinositol (GPI) anchored molecule, although it can also occur as a soluble form (Alemany, R. et al. (1993) J. Cell Sci. 104 ( Pt 4):1155-1162, Brown,J.P., et al. (1981) Proc. Natl, Acad. Sci. U. S. A 78:539-543, Food, M.R. et al. (1994). J. Biol. Chem. 269:3034-3040). Although expression of melanotransferrin is coincident with TfR1 in human brain capillary endothelium Rothenberger, S., et al. (1996) Brain Res. 712:117-121), neither human lactoferrin nor melanotransferrin are able to bind to TfR1 (Wally J, et al. (2006). J Biol Chem, 281, 24934-24944 and Kawabata et al. (2004) Br. J. Haematol. 127:464-473.). Melanotransferrin is also unable to bind to TfR2 and Kawabata et al. (2004) Br. J. Haematol. 127:464-473.

Different animal transferrins may have different receptor binding capability, for example Kawabata et al. (2004) Br. J. Haematol. 127:464-473 demonstrate that bovine human serum transferrin is able to interact with TfR1 but not TfR2.

Method 1 describes a method for selection of amino acid residues and regions on transferrin suitable for modification to introduce a free thiol group which when conjugated with a bioactive molecule reduces binding to transferrin receptor 1. Method 1 is in particular with reference to transferrin and transferrin receptor 1, however, the skilled person will appreciate that the teaching applies likewise to other members of the transferrin family, such as lactoferrin and melanotransferrin and to alternative transferrin receptors.

### Model/Method 2 Selection of amino acid residues and regions on transferrin surface-suitable for modification to introduce a free thiol group

Modifications to the transferrin molecule that alter transferrin receptor binding capacity by altering the amino acid sequence are known to the art. For example Cheng, Y., et al (2004) Cell 116:565-576 discloses a transferrin (H349A, D356A, and E357A) triple mutant that shows reduced binding to TfR expressing K562 cells. In addition US7176278 teaches modifications transferrin fusion proteins for extended serum half-life containing a transferrin moeity modified to reduce or inhibit glycosylation, iron binding and/or transferrin receptor binding all of which are herein incorporated by reference.

The iron binding sites on human serum transferrin are Asp-63, Tyr-95, Tyr-188, His-249, Asp-392, Tyr-426, Tyr-517, His-585. Transferrin muteins with altered metal binding capacity are also known Grady, J. K, et al. (1995). Biochem J 309 ( Pt 2): 403-10, He, Q.Y., et al. (2000) Biochemistry 39 p1205-1210, He,Q.Y., et al, (1997) Biochemical Journal 328 p439-445, and He,Q.Y., et al (1997) Biochemistry 36 p14853-14860. He,Q.Y., et al (1997) Biochemistry 36:14853-14860 describe D63S, D63C, G65R, Y95F, Y188F, K206Q, H207E, H249E, H249Q, K296E and K296Q hTf/2N transferrin variants which have altered iron binding when compared with that of the hTf/2N transferrin. The mutation Y188F of the hTf/2N transferrin completely abolishes iron binding. Mason, A.B., et al (2005) Biochemistry 44:8013-8021 describes a Y517F mutation and a Y426F mutation (located in the C-lobe) which are essential for the binding of iron. Such 'iron binding' mutants would also be expected to have no or reduced binding to the transferrin receptor since the binding capacity of holo-transferrin is 100-fold higher than that of apo-transferrin.

A synergistic carbonate anion is tethered by critical threonine (Thr 120 in N-lobe, and Thr-452 in C-lobe) and arginine (Arg 124 in N-lobe, and Arg-456 in C-lobe) residues. There is an the absolute dependence of iron binding on concomitant binding of this synergistic anion. Thus carbonate binding mutants would also be expected to have altered metal binding. A list of ligand mutants of human STF and human LTF is given in Lambert, L.A et al (2005) Comparative Biochemistry and Physiology Part B: Biochemistry and Molecular Biology 142 p129-141.
The full length, C-lobe/domain or N-lobe/domain transferrins with mutations in one or more of the amino acids responsible for iron or carbonate co-ordination would also be expected to have reduced TfR1 binding.

The coordinating iron binding residues for lactoferrin are Asp-61, Tyr-93, Tyr-193, and His-254 in the N-lobe and the corresponding Asp-396, Tyr-436, Tyr-529, and His-598 in the C-lobe. Ward, P.P., et al. (1996) J. Biol. Chem. 271:12790-12794 describe a lactoferrin variants which abolish the iron-binding ability of this protein.

In a second aspect to the invention thiotransferrin variants may be engineered to contain one or more free thiol groups by method 2 (described below). Alteration of the transferrin amino acid sequence to introduce one or more free thiol groups by introduction of unpaired cysteine residues on transferrin surface may or may not modify binding to one or more of the transferrin receptors, however when the thiotransferrin is conjugated with a bioactive molcule the conjugated thiotransferrin has reduced receptor binding activity to one or more of the transferrin receptors.

In further embodiements thiotransferrin variants already engineered to contain one or more free thiol groups by method 2 (described below) may be further modified to introduce modifications known to the art which reduce or abolish binding to one or more transferrin receptors.

Thiotransferrin muteins may be altered in their natural binding to one or more transferrin receptor. These thiotransferin muteins may also be altered in their natural binding to metal ions and coordinating anion.

### Model/Method 2 Selection of amino acid residues and regions on transferrin surface suitable for modification to introduce a free thiol group - Step 1

This method for indentifying position on the surface of the transferrin molecule that are suitable for introducing a free cysteine residue is described in the co-pending application PCT/EP2008/060482 and applies also for this application.

Based on this analysis described in details in PCT/EP2008/060482 the following residues can be identified based on the HST 3D structure, the Accessibility and RMSF analysis performed and the mapping analysis performed above, the preferred regions for introduction of the one or more cysteine residues with a free thiol groups may be taken as all the loops, sheets and helices i.e. V1-T5, E13-H25, M26-S36, K42-S44, Y85-T93, K103-Q108, L112-K115, T120-W128, L139-P145, F154-G156, A159-F167, P168-L170; P175-C177, G178-F186, G187-K196, D197-D201, I210-N213, L214-N216, K217-R220, D221-Q222, L226-P234, S255-K259, E260-H273, F274-H300, V305-D310, Y317-E328, G329- P341, C402-N417, C418-D420, K434-T440, W441-N443, L444-G446, Y468-K470, I471-R475, A485-S501, G502-N504, L505-Y515, G516-V526, T537-Q540, N541-D548, P549-A551, K552-N555, D558-Y559, C563-T567, R568-P570, E572-N576, E594-F608, G609-V636, L641-T646, R663-S668, S669-R677. Particularly preferred ranges include all the loops identified in table 1 of PCT/EP2008/060482 and so excludes the sheets and helices], i.e. V1-K4, M26-P35, K42-S44, Y85-T93, K103-Q108, L112-K115, T120-W128, L139-P145, F154-S155, A159-F167, G178-F186, D197-D201, L214-N216, D221-Q222, L226-P234, S255-K259, F274-H300, V305-D310, G329- P341, C402-N417, K434-T440, L444-G446, I471-R475, A485-S501, L505-Y515, N541-D548, K552-N555, D558, C563-T567, G609-V636, L641-T646, R663-S668.

For a transferrin family protein with two lobes, the lobes may be aligned as described in J. Wally et al., Journal of Biological Chemistry, 281 (34), 24934-24944 (2006), and residues may be selected which meet the criteria in both lobes. Most preferred ranges cover loops which were identified in both the N-Iobe and C-Iobe in Table 1 of PCT/EP2008/060482 i.e. V1-K4, K103-Q108, L112-K115, L139-P145, A159-F167, G178-F186, F274-H300, V305-D310, G329-P341, K434-T440, L444-G446, I471-R475, A485-S501, L505-Y515, G609-V636, L641-T646.

Most especially preferred positions for introduction of the one or more cysteine residues with a free thiol groups is defined as the amino acids identified in table 1, i.e. V1, P2, D3, K4 T5, H14, Q20, S21, D24, K27, S28, V29, P31, S32, D33, A43, E89, D104, G106, G114, L122, G123, P145, S155, D163, T165, D166, P168, P175, G176, G178, C179, S180, T181, L182, Q184, F187, S189, D197, G198, E212, A215, N216, A218, D221, D229, G257, N268, D277, K278, K280, E281, S287, P288, H289, K291, S298, P307, L326, T330, P335, T336, N413, S415, D416, D420, K434, S435, A436, S437, D438, D442, N443, G446, N469, N472, G487, K489, D491, S501, G502, L503, N510, T518, P539, Q540, G543, G544, K545, P547, D548, P549, K552, N553, N555, D558, D565, T567, P570, N576, A595, S610, N611, V612, T613, D614, S616, G617, T626, D634, D643, S666, T667, S669.

Method 2 of this the invention is described in particular with reference to transferrin, however, the skilled person will appreciate that the teaching applies likewise to other members of the transferrin family, such as lactoferrin and melanotransferrin.

### Alteration of transferrin amino acid sequence

A free thiol group may be introduced into the transferrin molecule by altering the amino acid sequence by substitution or insertion at a position selected as described above. Thus, the selected residue may be substituted with cysteine (the amino acid length is unchanged), or cysteine may be inserted at the N- or C-terminal side of the selected residue (the amino chain length is increased), or one or more adjacent residues including the selected residue may be substituted with cysteine (the amino acid chain length is reduced). Multiple alterations may be made to the polypeptide.

Alternatively, one of the cysteine residues present in the transferrin molecule (38 in the case of HST) may be selected, and the selected cysteine may be deleted or may be substituted with a different amino acid, particularly Ser, Thr, Val or Ala, cysteine residues in the regions selected above correspond to C19, C158, C161, C177, C179, C194, C227, C331, C339, C402, C418, C474, C495, C448, C506, C523, C563, C596, C615, C620, C665. The selected cysteine residue may in particular correspond to C227, C241, C474, C577, C563 or C665.

Alteration of the transferrin amino acid sequence to introduce one or more free thiol groups by introduction of unpaired cysteine residues on transferrin surface may or may not modify binding to one or more of the transferrin receptors, however when the thiotransferrin is conjugated with a bioactive molcule the conjugated thiotransferrin has binding activity of less than 5% to one or more of the transferrin receptors.

### Production of thiotransferrin

Detailed description regarding production of variants of transferrin includeing thiotransferrin is disclosed in the co-pending international patent application PCT/EP2008/060482. The teaching therein also applies for the present application and is included by reference.

### Formulation of thiotransferrin or conjugate

Detailed teaching concerning the formulation of thiotransferrin or conjugates thereorf can be found in the international patent application PCT/EP2008/060482 and is incorporated herein by reference.

### Nanoparticle Formulation

A problem of current conjugation strategies is maintaining both the pharmacological and immunological activity of the bioactive-targeting ligand conjugate. There is likely to be a maximum number of protein targeting ligand /bioactive moieties possible for conjugation at which point the targeting ligand does not retain its biological activity. Preferably the conjugation a bioactive compound to thiotransferrin should not affect the biological activity of the thiotransferrin).

Liposomes and nanoparticles offer an exciting alternative allowing entrapment of bioactive compounds into liposomes and nanoparticles. They provide a mechanism for enhanced delivery of drugs bioactive compounds, or uptake by, target cells and/or a reduction in the toxicity of the free bioactive to non-target organs ideally resulting in increased therapeutic index and/or reduced side effects. In addition, many solvent-based formulations required for some bioactive compounds (e.g. taxanes) are associated with toxicity which limits the dose given. Liposomes and nanoparticles delivery may also be advantageous for such bioactive compounds, since they would allow larger amounts of the bioactive compound to be delivered whilst avoiding some of the toxicities of solvent-based formulations (Advanced Drug Delivery Reviews (2008) 60, 8, p876-885.

Methods for attaching targeting ligands to liposomes and nanoparticles are known to the art (reviewed in Journal of Pharmaceutical Sciences Vol 93 p1980-1992). Both non-covalent and covalent methods for attachment have been described. Covalent reactions appear to be favorable, because covalent linkage is more stable than noncovalent methods. Lipids for the covalent or non-covalent attachment of proteins, peptides, or drugs to the liposome surface are available commercially (for example Avanti Polar Lipids Inc Alabaster, Alabama, USA. There are 3 major classes of functionality: conjugation through disulfide or thioether formation, amide bond formation, or biotin/streptavidin binding.

A number of methods relying on covalent coupling ligands to the surface of liposomes via thioether bonds have been described, most commonly utilizing the high efficient reaction of maleimide with thiol groups. Functionalized lipid anchors commonly added to liposomes include, but are not limited those containing maleimide such as N-[4-(p-maleimidophenyl) butyramide]-PE (N-MPB]-PE) or N- [4-(p-maleimidomethyl) cyclohexane-carboxamide) (MCC-PE) which allow convenient covalent coupling of the targeting moiety via a stable thioether bond (Martin & Papahadjopoulos (1982) J. Biol. Chem. 257, 286- 288).

Similarly materials for the formation of nanoparticles, including but are limited to Poly(lactic acid) (PLA), poly(lactic-co-glycolic acid) (PLGA), and COOH-PLA are commercially available and may be functionalized with maleimide or other known chemistries according to known literature for nanoparticle formation

Another convenient way for covalent coupling of ligands to liposomes involves conjugation of two thiols to form a disulfide; however under the reductive conditions in serum this is not ideal, thus more stable conjugation chemistries involving one free thiol group are preferred. Chemistries such as (PDP-PE) allow covalent coupling via a disulfide bond. Usually modification of the ligand to introduce a free thiol group or a functionalized linker is required; an advantage of thioalbumin/thiotransferrin is that no ligand modification is required.

Frequently thiol groups are not present in proteins, or are not present in sufficient amounts or at the desired location. Thus, most cases of covalent coupling of one of more ligands to a liposome via thioether or disulfide bonds require the use of heterobifunctional cross linking agents (described in conjugation section). Some heterobifunctional cross linking agents (such as SPDP and SATA) require a de-protection step ( Thiotransferrin described in this invention overcomes this additional processing.In a preferred embodiment transferrin variants according to the invention comprising at least two cysteine residue with a free thiol group located on the surface of the polypeptide may be used for the preparation of nanobodies where one or more cysteine residues with a free thiol group on the surface of the polypeptide may be used in the formation of the nanobodies and at least one cysteine residue with a free thiol group located on the surface of the polypeptide is used for conjugation.

Alternatively thiotransferrin could be conjugated to liposomes or nanoparticles by other chemistries, known to the art. For example, Thiotransferrin could be attached by an amide bond using a functionalised lipid anchor with either amine or carboxyl functional groups (examples include DSPE-PEG-COOH) which reacts with the primary amine of the ligand. Direct cross linking between primary amines and the surface of liposomes has also been described. The one or more free thiol groups of thioalbumin/thiotransferrin would then be available for conjugation to another bioactive compound. Nanoparticles may also be prepared using a mixture of traneferrin and transferrin variants according to the invention.

Another common problem for conjugates of bioactive compounds to targeting moieties is maintaining the binding activity of to the targeting moiety to their cognate receptors. Thiotransferrin described in this invention overcomes this problem and also may allow design of a variant with good orientation on the nanoparticle or liposome surface.

### Therapeutic compounds

The thiotransferrin according to the invention can be conjugated to various therapeutic compounds. Examples of therapeutic compounds can be found in PCT/EP2008/060482 and these examples also apply for the present invention.

### Alignment and identity

The HST variant may have at least 40 % identity with SEQ ID NO: 1, particularly at least 45%, 50%, 55%, 60%, 65%, 70%, 75 %, at least 80 %, at least 85 %, at least 90 %, at least 95 %, at least 97 %, at least 98 % or at least 99 % identity.

The Lactoferrin variant may have at least 40 % identity with SEQ ID NO: 2, particularly at least 45%, 50%, 55%, 60%, 65%, 70%, 75 %, at least 80 %, at least 85 %, at least 90 %, at least 95 %, at least 97 %, at least 98 % or at least 99 % identity.

The melanotransferrin variant may have at least 40 % identity with SEQ ID NO: 3, particularly at least 45%, 50%, 55%, 60%, 65%, 70%, 75 %, at least 80 %, at least 85 %, at least 90 %, at least 95 %, at least 97 %, at least 98 % or at least 99 % identity.

Alignments of a number of transferrin family proteins with HST (SEQ ID NO: 1) can be found in PCT/EP2008/060482 figure 6-9. A structural alignment of human lactoferrin with HST is described by J. Wally et al., Biometals (2007) 20:249-262. These alignments can be used to identify regions and amino acid residues corresponding to those in HST selected as described above. For other lactoferrin family proteins, primary sequence alignment can be performed by a number of procedures known to the art.

### Ligand binding

### Receptor binding

In some embodiments the polypeptide may include insertions, deletions and substitutions, either conservative or non-conservative, where such changes do not substantially reduce the useful ligand-binding, immunological or receptor binding properties of transferrin. The polypeptide may have at least 5%, 10%, 15%, 20%, 30%, 40% or 50%, 60%, 70%, at least 80%, 90%, 95%, 100%, 105% or more of a human transferrin's receptor binding activity, mole for mole.
In some embodiments the polypeptide may include insertions, deletions and substitutions, either conservative or non-conservative, where such changes substantially reduce the useful ligand-binding, immunological or receptor binding properties of transferrin. In some embodiments the unconjugated thiotransferrin moeity will bind one or more transferrin receptors with a capacity of less than 5%. The binding to other transferrin receptors may or may not be altered.
In some embodiments polypeptide may have increased affinity for the one or more transferrin receptors or altered binding or reduced release of iron (Adams et a/., 2003 J Biol Chem, 278 (8), 6027-33; Baker et al., 2007 Acta Crystallogr D Biol Crystallogr, 63 (Pt 3), 408-14; He and Mason, 2002 Molecular and Cellular Iron Transport, 5-123; Mason et al., 2005 Biochemistry, 44 (22), 8013-21. Yoon,D.J., et al (2008). Genetically engineering transferrin to improve its in vitro ability to deliver cytotoxins. J. Control Release 133 (3) p178-84 -Epub 2008 Oct 21).

When thiotransferrin polypeptide of this invention is conjugated to a bioactive compound, the thiotransferrin-bioactive conjugate has a binding capacity of less than 5% to one or more transferrin receptors compared with the binding capacity of unmodified transferrin.. The binding to other transferrin receptors may or may not be altered.
The polypeptide may display modified (e.g. reduced) glycosylation, such as, but not limited to reduced N-linked glycosylation or reduced O-linked glycosylation. The N-linked glycosylation pattern of a transferrin molecule can be modified by adding/removing amino acid glycosylation consensus sequences such as N-X-S/T, at any or all of the N, X, or S/T position. As a preferred example may O-linked glycosylation of HST molecule be modified by adding/removing/substituting serine at position 32.

### Transferrin Receptor Binding assays

The receptor binding capability of the recombinant transferrin variants can be determined by Surface Plasmon Resonance (SPR) analysis. The binding activity of a transferrin sample to a transferrin receptor can be measured using surface plasmon resonance (SPR) a non-invasive optical technique in which the SPR response is a measure of change in mass concentration at the detector surface as molecules bind or dissociate. A sample is sent onto the surface of the sensor chip via a micro flow system at a constant flow rate. In this analysis, if the transferrin sample is able to bind to the Transferrin Receptor the mass on the surface sensor chip is increased due to binding between Transferrin Receptor and Transferrin molecules creating a surface plasmon wave, and a shift of the SPR signal proportional to the binding quantity can be detected as a change in the resonance unit (RU). A response of 1 RU is equivalent to change in a surface concentration of about 1 pg .mm⁻².

The binding capacity of human serum transferrin to human trabnsferrin receptor 1 may be measured by SPR. Biacore sensor chips for interaction analysis between transferrin and the transferrin receptor 1 can be prepared by first immobilizing Transferrin receptor antibody prior to addition of the transferrin. Specifically, anti-Transferrin receptor 1 (anti-TfR1) antibody was immobilized to the CM5 sensor chip surface (GE Healthcare catalogue number BR-1000-14) using amine coupling chemistry at 25 °C. The carboxymethylated dextran surface on a CM5 sensor chip flow cell were converted to active succinamide esters by the addition of *N*-hydroxysuccinimide:*N*-ethyl-N'-(dimethylaminopropyl) carbodiimide (NHS:EDC).

The (Transferrin) receptor specific binding can be confirmed by concurrently preparing a sensor chip having an immobilized protein other than transferrin receptor and deducting the change in the resonance unit when the sample specimen is allowed to flow onto this chip to exclude a so-called bulk effect by a solvent or the like. The Anti-TfR 1 antibody (AbD Serotec catalogue number MCA1148) is diluted to 10 µg.mL⁻¹ in 10mM sodium acetate pH 5.0 (GE Healthcare catalogue number BR-1003-50) and injected overflow cell 2 only. Whereas 50 µL of the transferrin receptor 1 (TfR1) (AbD Serotec catalogue number 9110-300) (diluted in HBS-EP (10 mM HEPES, 150 mM NaCl, 3 mM EDTA, 0.005 % surfactant P-20, pH 7.4) to 10-20 µg.mL⁻¹) is injected over both flow cells. Excess ester groups on sensor chip surface are deactivated using ethanolamine hydrochloride (1 M pH 8.5).

HBS-EP is used as running buffer and dilution buffer for interaction analysis. Purified transferrin 50 µL is injected over both flow cells. Replicates should carried out to ensure reproducibility, The prepared Biacore sensor chip surface is regenerated between addition purified recombinant transferrin variants by 6-12 s injections of 10 mM sodium acetate pH 4.5 (GE Healthcare catalogue number BR-1003-50) between sample injections. Up to three injections are made, as required until baseline is restored.

A thiotransferrin is considered to bind to a transferrin receptor if it has at least 5 % of the receptor binding capacity of the corresponding polypeptide without cysteine insertion.

Those skilled in the art will appreciate that the binding capacity of human serum transferrin to other transferrin receptors may also be measured by SPR. West,A.P., et al (2000) J. Biol. Chem. 275 p38135-38138 teaches the skilled person how to measure interactions of transferrin with transferrin receptor 2. Koryraki,R., et al (2001) Proc. Natl. Acad. Sci 98 p12491-12496 teaches the skilled person how to measure interactions of transferrin with cubulin.

### Iron binding

Thiotransferrin mutants may have altered ability to bind or release iron and/or altered recycling time. Teaching concerning the determination of iron binding capability of transferrin variant can be found in the co-pending application PCT/EP2008/060482.

### Conjugation

Conjugation of the transferrin variants according to the invention with a bioactive compound can be performed as disclosed in PCT/EP2008/060482 and this teaching also apply for the present invention.

### EXAMPLES

Preparation of transferrin variants, conjugates thereof, determination of the iron binding properties of such variants as well as modelling of polypeptides for identification of suitable residues on the surface has been exemplified in PCT/EP2008/060482. These examples are also relevant for this application.

### MATERIALS AND METHODS

Chemicals used in the examples below are provided from Merck unless otherwise stated.

### Urea gel electrophoresis

Urea gel electrophoresis is performed using a modification of the procedure of Makey and Seal (Monthony et al, 1978, Clin. Chem., 24, 1825-1827; Harris & Aisen, 1989, Physical biochemistry of the transferrins, VCH; Makey & Seal, 1976, Biochim. Biophys. Acta., 453, 250-256; Evans & Williams, 1980, Biochem. J., 189, 541-546) with commercial minigels (6% homogeneous TBE Urea, Invitrogen). Samples containing approximately 10 µg protein are diluted 1:1 in TBE-Urea sample buffer (Invitrogen), separated at 180 V for 550 to 600 Vh and stained with GelCode^{®} Blue reagent (Pierce). Apo-transferrin is prepared by dialysis against 0.1 M citrate, 0.1 M acetate, 10 mM EDTA pH 4.5. Solutions are filtered (0.22µm), concentrated to 10 mg/ml using a Vivaspin polyethersulphone 10,000 NMWCO centrifugal concentrator and diafiltered against 10 volumes water followed by 10 volumes of 0.1 M HEPES, 0.1 M NaHCO₃ pH 8.0. Samples are recovered from the concentrator with a rinse and made up to a final concentration of 5 mg/ml. Reconstituted holo-transferrin is prepared from this solution by addition of 10 µl 1mM FeNTA (prepared freshly as an equimolar solution of ferric chloride in disodium nitrilotriacetic acid) to a 50 µl aliquot and allowed to stand for 10 minutes to permit CO₂ dissolution for completion of iron binding before electrophoretic analysis. This technique separates four molecular forms with different iron loadings namely (in order of increasing mobility) apo-transferrin, C-lobe and N-lobe bound monoferric transferrins and holo-transferrin. Separation of the four forms of transferrin is believed to be due to partial denaturation in 4-6M urea; where iron binding in any lobe causes a change in conformation resulting in increased resistance to denaturation. Thus the presence of iron in a lobe results in a more compact structure with higher electrophoretic mobility. Since the N-lobe has fewer disulphide bonds than the C-lobe (8 versus 11 respectively) it unfolds further in the absence of iron, making the monoferric form with iron bound to the C-lobe the least mobile.

### Free thiol assay

The number of free thiols on a protein can be determined spectrophotometrically using Ellman's reagent. Ellman's reagent (5'5'-dithio-bis(2-nitronenzoic acid) (DTNB)) is an aromatic disulphide which reacts with thiol groups to form a mixed disulphide of the protein and one mole of 2-nitro-5-thio-benzoate (NTB) (per mole of protein sulphidyl group). Formation of NTB can be monitored by measuring absorbance at 412nm. The molar absorbance coefficient is 13,600 M-1 cm-1

The free thiol assay using Ellman's reagent can be used to determine number of free thiol groups for proteins. To determine the number of free thiol groups the sample protein was diluted to a known concentration (e.g. 10 mg/mL) in 7.4 mM phosphate buffer pH 7.4 in a final volume of 1 mL. The protein solution (600 mL) was treated with 5,5'-dithiobis(2-nitrobenzoic acid) (DTNB) prepared freshly in 37 mM phosphate buffer, pH 8.3. Following 45 minutes at room temperature in the presence of EDTA, the absorbance of the solution was measured at 412 nm against reagent blanks and number of reacting sulfydryls calculated using E 412 =13600 M⁻¹cm⁻¹.

### EXAMPLE 1: TRANSFERRIN RECEPTOR BINDING

Transferrin receptor binding was determined as follows.

### ⁵⁵Fe uptake competition in erythroleukemic K562 cells

Human plasma-derived apo-transferrin (Calbiochem 616419, tissue culture grade, pyrogen-free) is supplied lyophilised from 10mM phosphate buffer, pH7.4. The transferrin mutein is expressed, purified and quantified as described above.

For iron-55 uptake from labeled diferric transferrin, K562 erythroleukemic cells, cultured in RPMI cell culture medium under standard conditions (bicarbonate-buffered, 5% (v/v) CO₂, antibiotics, 10% (v/v) fetal calf serum) are washed with serum-free medium containing HEPES-buffer and 1 mg/ml of bovine serum albumin and used at a concentration of 10 million cells/ml in this medium. The samples tested are prepared as equimolar concentrations of apo-transferrin mutein. Apo-transferrin mutein is loaded with iron according to a standard procedure (Bates and Schlabach, J Biol Chem, 248, 3228-3232, 1973) using ferric nitrilotriacetate as iron source. Typically 50µl of 1 M NaClO₄ is added to 450µl of the transferrin mutein stock-solution (pH is alkaline to neutral). The ⁵⁵Fe -NTA-loading-buffer is prepared by mixing 8.5µl 50mM NTA (pH 8.25), 18.9µl 0.1M Tris, 98.8µl Millipore-purified water and 7.5µl ⁵⁵FeCl₃ in 0.5 N HCl (NEN products). 500µl of transferrin mutein is carefully mixed (dropwise) with the ⁵⁵Fe -NTA-loading-buffer and then 310µl of 5mM Hepes. NaOH/0.1M NaClO₄ is added. The mixture is incubated at 4°C for 60 min following which it is deslated on a PD-10 column (containing Sephadex G-25) and dialysed. The PD-10 column is equilibrated with 5ml of equilibration buffer (5mM Hepes/NaOH, 0.1M NaClO₄, 0.1 g BSA (Amresco)) then washed three times with 5ml of of wash buffer (5mM Hepes/NaOH, 0.1M NaClO₄). Iron (⁵⁵Fe)-loaded transferrin mutein is loaded on to the column and eluted with elution buffer (5mM Hepes/NaOH, 0.1M NaClO₄). The eluted iron (⁵⁵Fe)-loaded transferrin mutein is dialysed at 4°C against 5mM Hepes, 0.15 mM NaCl, pH 7.4.

Increasing concentrations of human plasma transferrin or the transferrin mutein sample (0, 25, 100, 200, 400, 800, 1600 nM), labeled with ⁵⁵Fe, are mixed with 25 µl of medium. The reaction is started by the addition of 300 µl of cell suspension. A second series of parallel experiments is carried out in the presence of a hundredfold excess of unlabeled diferric transferrin to account for unspecific binding. After 25 minutes at 37°C the reaction is stopped by immersion into an ice-bath, three aliquots of 60µl of cell suspension are transferred to new tubes and the cells are centrifuged in the cold and again after addition of an oil layer of diethylphtalate/dibutylphthalate. The supernatant is removed, the cell pellet transferred into a counter vial and lysed with 0.5 M KOH + 1% (v/v) Triton X-100. The lysates are neutralized with 1 M HCl after overnight lysis, mixed with Readysolv Scintillation cocktail and counted in the Packard Liquid Scintillation Counter. The results are typically presented as fmol ⁵⁵Fe/million cells.

### Competition of iron uptake into human K562 cells by transferrin mutein and radiolabelled plasma transferrin

Additionally a competition assay is performed using a constant concentration of ⁵⁵Fe-loaded plasma transferrin (100nM) with non-radioactive labelled holotransferrin mutein in concentrations ranging from 0 to 1600nM (0, 25, 100, 200, 400, 800, 1600 nM). Iron (⁵⁵Fe)-loaded plasma transferrin is prepared by described above. K562 cells are added to the incubation mixture to give a cell density of 10⁶cells/ml. RPMI-medium containing 0.1 % (w/v) of bovine serum albumin and 10mM Hepes is used for dilution of transferrins and cells. After 25 minutes at 37°C the reaction is stopped by immersion into an ice-bath, three aliquots of 60µl of cell suspension are transferred to new tubes and the cells are centrifuged in the cold and again after addition of an oil layer of diethylphtalate/dibutylphthalate. The supernatant is removed, the cell pellet transferred into a counter vial and lysed with 0.5 M KOH, 1% (v/v) Triton X-100. The lysates are neutralized with 1M HCl after overnight lysis, mixed with Readysolv scintillation cocktail and counted in the Packard Liquid Scintillation Counter. The results are presented as fmol ⁵⁵Fe/million cells.

A thiotransferrin is considered to bind to a transferrin receptor it has at least 5 % of the receptor binding capacity of the corresponding polypeptide without cysteine insertion

K562 cells are known to express both the transferrin receptor 1 and the transferrin receptor. If desired the method disclosed in this example may be conducted using a cell line that expresses only one of the tranferrin receptors. A K562 cell line which does not express TfR2 is available see Calzolari A, et al Blood Cells Mol Dis. 2009 Jan-Feb;42(1):5-13. Epub 2008 Nov 18.

### EXAMPLE 2: TRANSFERRIN RECEPTOR BINDING CAPABILITY OF RECOMBINANT THIOTRANSFERRIN VARIANTS COMPARED TO RECOMBINANT TRANSFERRIN (S415A, T613A)

The receptor binding capability of the recombinant transferrin variants was assessed by Surface Plasmon Resonance (SPR) analysis. The binding activity of a transferrin sample to transferrin receptor can be measured using surface plasmon resonance (SPR) a non-invasive optical technique in which the SPR response is a measure of change in mass concentration at the detector surface as molecules bind or dissociate. A sample is sent onto the surface of the sensor chip via a micro flow system at a constant flow rate. In this analysis, if the transferrin sample is able to bind to the TfR the mass on the surface sensor chip is increased due to binding between TfR and Tf molecules creating a surface plasmon wave, and a shift of the SPR signal proportional to the binding quantity can be detected as a change in the resonance unit (RU). A response of 1 RU is equivalent to change in a surface concentration of about 1 pg .mm⁻².

Biacore sensor chips for interaction analysis between transferrin and the transferrin receptor 1 were prepared by first immobilizing Transferrin receptor 1 antibody prior to addition of the transferrin. Specifically, anti- Transferrin receptor (anti-TfR1) antibody was immobilized to the CM5 sensor chip surface (GE Healthcare catalogue number BR-1000-14) using amine coupling chemistry at 25 °C. The carboxymethylated dextran surface on a CM5 sensor chip flow cell were converted to active succinamide esters by the addition of *N*-hydroxysuccinimide:*N-*ethyl-N'-(dimethylaminopropyl) carbodiimide (NHS:EDC).

The (Transferrin) receptor specific binding can be confirmed by concurrently preparing a sensor chip having an immobilized protein other than transferrin receptor and deducting the change in the resonance unit when the sample specimen is allowed to flow onto this chip to exclude a so-called bulk effect by a solvent or the like. The Anti-TfR1 antibody (AbD Serotec catalogue number MCA1148) was diluted to 10 µg.mL⁻¹ in 10mM sodium acetate pH 5.0 (GE Healthcare catalogue number BR-1003-50) and injected over flow cell 2 only. Whereas 50 µL of the transferrin receptor 1 (TfR1) (AbD Serotec catalogue number 9110-300) (diluted in HBS-EP (10 mM HEPES, 150 mM NaCl, 3 mM EDTA, 0.005 % surfactant P-20, pH 7.4) to 10-20 µg.mL⁻¹) was injected over both flow cells. Excess ester groups on sensor chip surface were deactivated using ethanolamine hydrochloride (1 M pH 8.5).

HBS-EP was used as running buffer and dilution buffer for interaction analysis.

Thiotransferrin muteins expressed, purified and quantified as described in PCT/EP2008/060482 were compared for transferrin receptor 1 binding capacity measured by surface plasmon resonance (SPR). Purified iron-loaded recombinant transferrin (S415A, T613A) or recombinant thiotransferrin (S28C, S415A, T613A), recombinant thiotransferrin (S32C, S415A, T613A), recombinant thiotransferrin (A215C, S415A, T613A), recombinant thiotransferrin (S415C, T613A), and recombinant thiotransferrin (S415A, N553C, T613A) purified by the first chromatographic step was diluted to 20 µg.mL⁻¹ and 50 µL injected over both flow cells. Replicates were carried out to ensure reproducibility, The prepared Biacore sensor chip surface was regenerated between addition purified recombinant transferrin variants by 6-12 s injections of 10 mM sodium acetate pH 4.5 (GE Healthcare catalogue number BR-1003-50) between sample injections. Up to three injections were made, as required until baseline was restored.

Samples of recombinant thiotransferrin (S28C, S415A, T613A), recombinant thiotransferrin (S32C, S415A, T613A), recombinant thiotransferrin (A215C, S415A, T613A), recombinant thiotransferrin (S415C, T613A) and recombinant thiotransferrin (S415A, N553C, T613A) purified by the first chromatographic step were compared to purified iron-loaded recombinant transferrin (S415A, T613A) for their ability to bind Transferrin receptor 1.

Transferrin variants described in this example were considered to bind to transferrin receptor 1, since they had at least 5 % of the transferrin receptor 1 binding capacity of the corresponding polypeptide without cysteine insertion.

A person skilled in the art would be able to devise similar SPR transferrin receptor binding assays to assess binding of thiotransferrin variants to other transferrin receptor proteins.

### EXAMPLE 3: BINDING OF CONJUGATED THIOTRANSFERRIN VARIANTS TO TRANSFERRIN RECEPTOR 1 (MEASURED BY SPR)

Thiotransferrin muteins and fluorescein conjugated thiotransferrin muteins expressed, purified and quantified as described in PCT/EP2008/060482 were compared for transferrin receptor 1 binding capacity measured by surface plasmon resonance (SPR).

Thiotransferrin (S28C, S415A, T613A) sample which has been conjugated with fluorescein was able to bind to transferrin receptor 1 (measured by SPR). Measurement of transferrin receptor binding by SPR analysis showed that modification of a selected residue such as serine 28 to a cysteine residue does not alter the gross structure of transferrin or prevent binding of the transferrin to transferrin receptor 1.

Similarly, fluorescein conjugated thiotransferrin (S415C, T613A) samples was able to bind to transferrin receptor 1 by SPR analysis in a manner equivalent to the unconjugated sample. Measurement of transferrin receptor 1 binding by SPR analysis showed that modification of a selected residue such as serine 415 to a cysteine residue does not alter the gross structure of transferrin or prevent binding of the transferrin to transferrin receptor 1.

This example demonstrates that when a bioactive molecule is conjugated to the thiotransferrin mutein (S32C, S415A, T613A) through the sulphur atom of the cysteine residue that the thiotransferrin mutein retains its ability to bind to transferrin receptor 1. Similarly, when a bioactive molecule is conjugated to the thiotransferrin (S415C, T613A) mutein through the sulphur atom of the cysteine residue that the thiotransferrin mutein retains its ability to bind to transferrin receptor 1. In some embodiments the unconjugated thiotransferrin molecule may have binding capacity of more than 5% to one or more transferrin receptor; however when the thiotransferrin molecule is conjugated with a bioactive compound it will have a binding capacity of less than 5% to one or more transferrin receptor.

**Table 3**

| **Sample** | **Total Iron Uptake** | | **Specific iron ptake** | | |
|---|---|---|---|---|---|
| | **Bmax (fmol⁵⁵Fe/10⁶ cells x 25min)** | **R² -** | **Bmax (fmol⁵⁵Fe/10⁶ cells 25min)** | **Kd x (nM trans-ferrin)** | **R²** |
| Plasma-derived transferrin | 4252±185 | 0.9376 | 3277±164 | 166±33 | 0.8997 |
| Transferrin (S415A, T613A) | 4046±128 | 0.9613 | 2829±117 | 130±23 | 0.9028 |
| Thiotransferrin (S28C, S415A, T613A), | 4211±133 | 0.9713 | 3023±105 | 209±27 | 0.9501 |
| Fluorescein conjugated thio-transferrin (S28C, S415A, T613A) | 3294±170 | 0.9093 | 2430±125 | 154±32 | 0.8820 |
| Thiotransferrin (S415C, T613A) | 3691±256 | 0.8938 | 2359±150 | 203±47 | 0.8389 |
| Fluorescein conjugated thio-transferrin (S415C, T613A), | 4165±216 | 0.9288 | 3246±217 | 189±47 | 0.8559 |

## Claims

1. A polypeptide which
a. has an amino acid sequence which is at least 40% identical to residues 1-679, 1-331, or 339-679 of SEQ ID NO: 1, residues 1-691 of SEQ ID NO: 2 or residues 1-738 of SEQ ID NO: 3,
b. comprises at least one cysteine residue with a free thiol group located on the surface of the polypeptide
c. has a reduced binding to one or more of the transferrin receptors when said polypeptide is unconjugated or conjugated to a bioactive molecule, compared with unmodified human serum transferrin.

2. The polypeptide of claim 1, where the reduced binding in c, corresponds to 5 % or less binding to one or more of the transferrin receptors compared with unmodified transferrin.

3. The polypeptide of claim 1 or 2, selected among:
a. A polypeptide that has an amino acid sequence that is at least 80% identical to residues 1-331 of SEQ ID NO: 1;
b. A polypeptide that is at least 80% identical to SEQ ID NO: 1 and comprises substitution of an amino acid with cysteine, insertion or deletion of cysteine at a position corresponding to any of T5-A10, Y45- A53, A54-A59, A64-Y71, L72-N76, L139-P145, S255-K259, V305-D310, Y317-E328, G329- P341, G329-P341, L347-S348, H349-S362, V363-K365, A371-T373, G502-N504, G609-C637 of SEQ ID NO:1;
c. A polypeptide that is at least 80% identical to SEQ ID NO:1 and comprises substitution of deletion of at least one of the positions: Asp-63, Tyr-95, Tyr-188, His-249, Asp-392, Tyr-426, Tyr-517 and His-585 of SEQ ID NO:1.

4. The polypeptide of claim 3, that is at least 80% identical to SEQ ID NO: 1 and comprises substitution of an amino acid with cysteine, insertion or deletion of cysteine at a position corresponding to any of T5, D47, A53, A54, N55, D69, L72, A73, P74, P140, E141, P142, K144, P145, S255, M256, G257, M309, D310, L326, R327, G329, T330, E333, P335, T336, L347, H349, H350, V360, N361, V363, E372, L503, N510, S616, G617 and T626 of SEQ ID NO:1.

5. The polypeptide of claim 4, that is at least 80% identical to SEQ ID NO: 1 and comprises substitution of an amino acid with cysteine, insertion or deletion of cysteine at a position corresponding to any of A73, P74, P142, L326 and V363 of SEQ ID NO:1.

6. The polypeptide according to any of the claims 1-5, having at least 95% sequence identity to a serum transferrin, particularly derived from a vertebrate, a mammal or human.

7. The polypeptide of any preceding claims which comprises substitution of an amino acid with cysteine, insertion or deletion of cysteine at a position corresponding to any of V1-T5, E13-H25, M26-S36, K42-S44, Y85-T93, K103-Q108, L112-K115, T120-W128, L139-P145, F154-G156, A159-F167, P168-L170; P175-C177, G178-F186, G187-K196, D197-D201, I210-N213, L214-N216, K217-R220, D221-Q222, L226-P234, S255-K259, E260-H273, F274-H300, V305-D310, Y317-E328, G329- P341, C402-N417, C418-D420, K434-T440, W441-N443, L444-G446, Y468-K470, I471-R475, A485-S501, G502-N504, L505-Y515, G516-V526, T537-Q540, N541-D548, P549-A551, K552-N555, D558-Y559, C563-T567, R568-P570, E572-N576, E594-F608, G609-V636, L641-T646, R663-S668 or S669-R677 of SEQ ID NO: 1.

8. The polypeptide claim 7, which comprises substitution, insertion or deletion of an amino acid with cysteine at a position corresponding to any of V1, P2, D3, K4 T5, H14, Q20, S21, D24, K27, S28, V29, P31, S32, D33, A43, E89, D104, G106, G114, L122, G123, P145, S155, D163, T165, D166, P168, P175, G176, G178, C179, S180, T181, L182, Q184, F187, S189, D197, G198, E212, A215, N216, A218, D221, D229, G257, N268, D277, K278, K280, E281, S287, P288, H289, K291, S298, P307, L326, T330, P335, T336, N413, S415, D416, D420, K434, S435, A436, S437, D438, D442, N443, G446, N469, N472, G487, K489, D491, S501, G502, L503, N510, T518, P539, Q540, G543, G544, K545, P547, D548, P549, K552, N553, N555, D558, D565, T567, P570, N576, A595, S610, N611, V612, T613, D614, S616, G617, T626, D634, D643, S666, T667 or S669 of SEQ ID NO: 1.

9. The polypeptide of claim 8, wherein the polypeptide has at least 95% sequence identity to residues 1-679, 1-331, or 339-679 of SEQ ID NO: 1, and the at least one cysteine residue is selected among C19, C158, C161, C177, C179, C194, C227, C331, C339, C402, C418, C474, C495, C448, C506, C523, C563, C596, C615, C620, C665.

10. The polypeptide of any preceding claims which further comprises at least one mutation that reduces N- or O-linked glycosylation.

11. The polypeptide claim 10, which compared to SEQ ID NO: 1 comprises a substitution of an amino acid at a position corresponding to S32, N413, S415, N611 or T613 to an amino acid which does not allow glycosylation at the position corresponding to S32, N413 or N611.

12. The polypeptide of any of the preceding claims, having at least 99% sequence identity to SEQ ID NO: 1 and comprises one or more mutations selected among: V1C, S28C, S32C, D104C, T165C, P175C, A215C, P288C, T336C, S415C, D146C, C171A, S415C+deletion of D416, S415A+insertion of C before D416, S501C, N553C, N611C, T613C, D643C and S28C+S415C

13. The polypeptide of any of the claims 1-10, having at least 95% sequence identity to SEQ ID NO: 2 and comprises the mutation S421C.

14. A polynucleotide which encodes the polypeptide of any of the claims 1-13,

15. A host cell, preferably a yeast cell, comprising a polynucleotide of claim 14.

16. A conjugate which comprises a bioactive compounds and a polypeptide according to any of the claims 1-12, wherein the bioactive compound is linked through the free thiol group of the cysteine residue of the polypeptide.

17. A composition comprising a conjugate of claim 16 and at least one pharmaceutically acceptable carrier or diluent.


SEQUENCE LISTING

<110> Novozymes Biopharma UK Ltd.

<120> Transferrin variants and conjugates

<130> 11522.000-EP

<160> 3

<170> PatentIn version 3.3

<210> 1
<211> 679
<212> PRT
<213> Homo Sapiens

<400> 1

Val Pro Asp Lys Thr Val Arg Trp Cys Ala Val Ser Glu His Glu Ala
1               5                   10                  15

Thr Lys Cys Gln Ser Phe Arg Asp His Met Lys Ser Val Ile Pro Ser
            20                  25                  30

Asp Gly Pro Ser Val Ala Cys Val Lys Lys Ala Ser Tyr Leu Asp Cys
        35                  40                  45

Ile Arg Ala Ile Ala Ala Asn Glu Ala Asp Ala Val Thr Leu Asp Ala
    50                  55                  60

Gly Leu Val Tyr Asp Ala Tyr Leu Ala Pro Asn Asn Leu Lys Pro Val
65                  70                  75                  80

Val Ala Glu Phe Tyr Gly Ser Lys Glu Asp Pro Gln Thr Phe Tyr Tyr
                85                  90                  95

Ala Val Ala Val Val Lys Lys Asp Ser Gly Phe Gln Met Asn Gln Leu
            100                 105                 110

Arg Gly Lys Lys Ser Cys His Thr Gly Leu Gly Arg Ser Ala Gly Trp
        115                 120                 125

Asn Ile Pro Ile Gly Leu Leu Tyr Cys Asp Leu Pro Glu Pro Arg Lys
    130                 135                 140

Pro Leu Glu Lys Ala Val Ala Asn Phe Phe Ser Gly Ser Cys Ala Pro
145                 150                 155                 160

Cys Ala Asp Gly Thr Asp Phe Pro Gln Leu Cys Gln Leu Cys Pro Gly
                165                 170                 175

Cys Gly Cys Ser Thr Leu Asn Gln Tyr Phe Gly Tyr Ser Gly Ala Phe
            180                 185                 190

Lys Cys Leu Lys Asp Gly Ala Gly Asp Val Ala Phe Val Lys His Ser
        195                 200                 205

Thr Ile Phe Glu Asn Leu Ala Asn Lys Ala Asp Arg Asp Gln Tyr Glu
    210                 215                 220

Leu Leu Cys Leu Asp Asn Thr Arg Lys Pro Val Asp Glu Tyr Lys Asp
225                 230                 235                 240

Cys His Leu Ala Gln Val Pro Ser His Thr Val Val Ala Arg Ser Met
                245                 250                 255

Gly Gly Lys Glu Asp Leu Ile Trp Glu Leu Leu Asn Gln Ala Gln Glu
            260                 265                 270

His Phe Gly Lys Asp Lys Ser Lys Glu Phe Gln Leu Phe Ser Ser Pro
        275                 280                 285

His Gly Lys Asp Leu Leu Phe Lys Asp Ser Ala His Gly Phe Leu Lys
    290                 295                 300

Val Pro Pro Arg Met Asp Ala Lys Met Tyr Leu Gly Tyr Glu Tyr Val
305                 310                 315                 320

Thr Ala Ile Arg Asn Leu Arg Glu Gly Thr Cys Pro Glu Ala Pro Thr
                325                 330                 335

Asp Glu Cys Lys Pro Val Lys Trp Cys Ala Leu Ser His His Glu Arg
            340                 345                 350

Leu Lys Cys Asp Glu Trp Ser Val Asn Ser Val Gly Lys Ile Glu Cys
        355                 360                 365

Val Ser Ala Glu Thr Thr Glu Asp Cys Ile Ala Lys Ile Met Asn Gly
    370                 375                 380

Glu Ala Asp Ala Met Ser Leu Asp Gly Gly Phe Val Tyr Ile Ala Gly
385                 390                 395                 400

Lys Cys Gly Leu Val Pro Val Leu Ala Glu Asn Tyr Asn Lys Ser Asp
                405                 410                 415

Asn Cys Glu Asp Thr Pro Glu Ala Gly Tyr Phe Ala Val Ala Val Val
            420                 425                 430

Lys Lys Ser Ala Ser Asp Leu Thr Trp Asp Asn Leu Lys Gly Lys Lys
        435                 440                 445

Ser Cys His Thr Ala Val Gly Arg Thr Ala Gly Trp Asn Ile Pro Met
    450                 455                 460

Gly Leu Leu Tyr Asn Lys Ile Asn His Cys Arg Phe Asp Glu Phe Phe
465                 470                 475                 480

Ser Glu Gly Cys Ala Pro Gly Ser Lys Lys Asp Ser Ser Leu Cys Lys
                485                 490                 495

Leu Cys Met Gly Ser Gly Leu Asn Leu Cys Glu Pro Asn Asn Lys Glu
            500                 505                 510

Gly Tyr Tyr Gly Tyr Thr Gly Ala Phe Arg Cys Leu Val Glu Lys Gly
        515                 520                 525

Asp Val Ala Phe Val Lys His Gln Thr Val Pro Gln Asn Thr Gly Gly
    530                 535                 540

Lys Asn Pro Asp Pro Trp Ala Lys Asn Leu Asn Glu Lys Asp Tyr Glu
545                 550                 555                 560

Leu Leu Cys Leu Asp Gly Thr Arg Lys Pro Val Glu Glu Tyr Ala Asn
                565                 570                 575

Cys His Leu Ala Arg Ala Pro Asn His Ala Val Val Thr Arg Lys Asp
            580                 585                 590

Lys Glu Ala Cys Val His Lys Ile Leu Arg Gln Gln Gln His Leu Phe
        595                 600                 605

Gly Ser Asn Val Thr Asp Cys Ser Gly Asn Phe Cys Leu Phe Arg Ser
    610                 615                 620

Glu Thr Lys Asp Leu Leu Phe Arg Asp Asp Thr Val Cys Leu Ala Lys
625                 630                 635                 640

Leu His Asp Arg Asn Thr Tyr Glu Lys Tyr Leu Gly Glu Glu Tyr Val
                645                 650                 655

Lys Ala Val Gly Asn Leu Arg Lys Cys Ser Thr Ser Ser Leu Leu Glu
            660                 665                 670

Ala Cys Thr Phe Arg Arg Pro
        675

<210>  2
<211>  691
<212>  PRT
<213>  Homo sapiens

<400>  2

Gly Arg Arg Arg Ser Val Gln Trp Cys Ala Val Ser Gln Pro Glu Ala
1               5                   10                  15

Thr Lys Cys Phe Gln Trp Gln Arg Asn Met Arg Lys Val Arg Gly Pro
            20                  25                  30

Pro Val Ser Cys Ile Lys Arg Asp Ser Pro Ile Gln Cys Ile Gln Ala

35                  40                  45

Ile Ala Glu Asn Arg Ala Asp Ala Val Thr Leu Asp Gly Gly Phe Ile
    50                  55                  60

Tyr Glu Ala Gly Leu Ala Pro Tyr Lys Leu Arg Pro Val Ala Ala Glu
65                  70                  75                  80

Val Tyr Gly Thr Glu Arg Gln Pro Arg Thr His Tyr Tyr Ala Val Ala
                85                  90                  95

Val Val Lys Lys Gly Gly Ser Phe Gln Leu Asn Glu Leu Gln Gly Leu
            100                 105                 110

Lys Ser Cys His Thr Gly Leu Arg Arg Thr Ala Gly Trp Asn Val Pro
        115                 120                 125

Ile Gly Thr Leu Arg Pro Phe Leu Asn Trp Ala Gly Pro Pro Glu Pro
    130                 135                 140

Ile Glu Ala Ala Val Ala Arg Phe Phe Ser Ala Ser Cys Val Pro Gly
145                 150                 155                 160

Ala Asp Lys Gly Gln Phe Pro Asn Leu Cys Arg Leu Cys Ala Gly Thr
                165                 170                 175

Gly Glu Asn Lys Cys Ala Phe Ser Ser Gln Glu Pro Tyr Phe Ser Tyr
            180                 185                 190

Ser Gly Ala Phe Lys Cys Leu Arg Asp Gly Ala Gly Asp Val Ala Phe
        195                 200                 205

Ile Arg Glu Ser Thr Val Phe Glu Asp Leu Ser Asp Glu Ala Glu Arg
    210                 215                 220

Asp Glu Tyr Glu Leu Leu Cys Pro Asp Asn Thr Arg Lys Pro Val Asp
225                 230                 235                 240

Lys Phe Lys Asp Cys His Leu Ala Arg Val Pro Ser His Ala Val Val
                245                 250                 255

Ala Arg Ser Val Asn Gly Lys Glu Asp Ala Ile Trp Asn Leu Leu Arg
            260                 265                 270

Gln Ala Gln Glu Lys Phe Gly Lys Asp Lys Ser Pro Lys Phe Gln Leu
        275                 280                 285

Phe Gly Ser Pro Ser Gly Gln Lys Asp Leu Leu Phe Lys Asp Ser Ala
    290                 295                 300

Ile Gly Phe Ser Arg Val Pro Pro Arg Ile Asp Ser Gly Leu Tyr Leu
305                 310                 315                 320

Gly Ser Gly Tyr Phe Thr Ala Ile Gln Asn Leu Arg Lys Ser Glu Glu
                325                 330                 335

Glu Val Ala Ala Arg Arg Ala Arg Val Val Trp Cys Ala Val Gly Glu
            340                 345                 350

Gln Glu Leu Arg Lys Cys Asn Gln Trp Ser Gly Leu Ser Glu Gly Ser
        355                 360                 365

Val Thr Cys Ser Ser Ala Ser Thr Thr Glu Asp Cys Ile Ala Leu Val
    370                 375                 380

Leu Lys Gly Glu Ala Asp Ala Met Ser Leu Asp Gly Gly Tyr Val Tyr
385                 390                 395                 400

Thr Ala Gly Lys Cys Gly Leu Val Pro Val Leu Ala Glu Asn Tyr Lys
                405                 410                 415

Ser Gln Gln Ser Ser Asp Pro Asp Pro Asn Cys Val Asp Arg Pro Val
            420                 425                 430

Glu Gly Tyr Leu Ala Val Ala Val Val Arg Arg Ser Asp Thr Ser Leu
        435                 440                 445

Thr Trp Asn Ser Val Lys Gly Lys Lys Ser Cys His Thr Ala Val Asp
    450                 455                 460

Arg Thr Ala Gly Trp Asn Ile Pro Met Gly Leu Leu Phe Asn Gln Ala
465                 470                 475                 480

Gly Ser Cys Lys Phe Asp Glu Tyr Phe Ser Gln Ser Cys Ala Pro Gly
                485                 490                 495

Ser Asp Pro Arg Ser Asn Leu Cys Ala Leu Cys Ile Gly Asp Glu Gln
            500                 505                 510

Gly Glu Asn Lys Cys Val Pro Asn Ser Asn Glu Arg Tyr Tyr Gly Tyr
        515                 520                 525

Thr Gly Ala Phe Arg Cys Leu Ala Glu Asn Ala Gly Asp Val Ala Phe
    530                 535                 540

Val Lys Asp Val Thr Val Leu Gln Asn Thr Asp Gly Asn Asn Asn Glu
545                 550                 555                 560

Ala Trp Ala Lys Asp Leu Lys Leu Ala Asp Phe Ala Leu Leu Cys Leu
                565                 570                 575

Asp Gly Lys Arg Lys Pro Val Thr Glu Ala Arg Ser Cys His Leu Ala
            580                 585                 590

Met Ala Pro Asn His Ala Val Val Ser Arg Met Asp Lys Val Glu Arg
        595                 600                 605

Leu Lys Gln Val Leu Leu His Gln Gln Ala Lys Phe Gly Arg Asn Gly
    610                 615                 620

Ala Asp Cys Pro Asp Lys Phe Cys Leu Phe Gln Ser Glu Thr Lys Asn
625                 630                 635                 640

Leu Leu Phe Asn Asp Asn Thr Glu Cys Leu Ala Arg Leu His Gly Lys
                645                 650                 655

Thr Thr Tyr Glu Lys Tyr Leu Gly Pro Gln Tyr Val Ala Gly Ile Thr
            660                 665                 670

Asn Leu Lys Lys Cys Ser Thr Ser Pro Leu Leu Glu Ala Cys Glu Phe
        675                 680                 685

Leu Arg Lys
    690

<210> 3
<211> 738
<212> PRT
<213> Homo sapiens

<400> 3

Met Arg Gly Pro Ser Gly Ala Leu Trp Leu Leu Leu Ala Leu Arg Thr
1               5                   10                  15

Val Leu Gly Gly Met Glu Val Arg Trp Cys Ala Thr Ser Asp Pro Glu
            20                  25                  30

Gln His Lys Cys Gly Asn Met Ser Glu Ala Phe Arg Glu Ala Gly Ile
        35                  40                  45

Gln Pro Ser Leu Leu Cys Val Arg Gly Thr Ser Ala Asp His Cys Val
    50                  55                  60

Gln Leu Ile Ala Ala Gln Glu Ala Asp Ala Ile Thr Leu Asp Gly Gly
65                  70                  75                  80

Ala Ile Tyr Glu Ala Gly Lys Glu His Gly Leu Lys Pro Val Val Gly
                85                  90                  95

Glu Val Tyr Asp Gln Glu Val Gly Thr Ser Tyr Tyr Ala Val Ala Val
            100                 105                 110

Val Arg Arg Ser Ser His Val Thr Ile Asp Thr Leu Lys Gly Val Lys
        115                 120                 125

Ser Cys His Thr Gly Ile Asn Arg Thr Val Gly Trp Asn Val Pro Val

130                 135                 140

Gly Tyr Leu Val Glu Ser Gly Arg Leu Ser Val Met Gly Cys Asp Val
145                 150                 155                 160

Leu Lys Ala Val Ser Asp Tyr Phe Gly Gly Ser Cys Val Pro Gly Ala
                165                 170                 175

Gly Glu Thr Ser Tyr Ser Glu Ser Leu Cys Arg Leu Cys Arg Gly Asp
            180                 185                 190

Ser Ser Gly Glu Gly Val Cys Asp Lys Ser Pro Leu Glu Arg Tyr Tyr
        195                 200                 205

Asp Tyr Ser Gly Ala Phe Arg Cys Leu Ala Glu Gly Ala Gly Asp Val
    210                 215                 220

Ala Phe Val Lys His Ser Thr Val Leu Glu Asn Thr Asp Gly Lys Thr
225                 230                 235                 240

Leu Pro Ser Trp Gly Gln Ala Leu Leu Ser Gln Asp Phe Glu Leu Leu
                245                 250                 255

Cys Arg Asp Gly Ser Arg Ala Asp Val Thr Glu Trp Arg Gln Cys His
            260                 265                 270

Leu Ala Arg Val Pro Ala His Ala Val Val Val Arg Ala Asp Thr Asp
        275                 280                 285

Gly Gly Leu Ile Phe Arg Leu Leu Asn Glu Gly Gln Arg Leu Phe Ser
    290                 295                 300

His Glu Gly Ser Ser Phe Gln Met Phe Ser Ser Glu Ala Tyr Gly Gln
305                 310                 315                 320

Lys Asp Leu Leu Phe Lys Asp Ser Thr Ser Glu Leu Val Pro Ile Ala
                325                 330                 335

Thr Gln Thr Tyr Glu Ala Trp Leu Gly His Glu Tyr Leu His Ala Met
            340                 345                 350

Lys Gly Leu Leu Cys Asp Pro Asn Arg Leu Pro Pro Tyr Leu Arg Trp
        355                 360                 365

Cys Val Leu Ser Thr Pro Glu Ile Gln Lys Cys Gly Asp Met Ala Val
    370                 375                 380

Ala Phe Arg Arg Gln Arg Leu Lys Pro Glu Ile Gln Cys Val Ser Ala
385                 390                 395                 400

Lys Ser Pro Gln His Cys Met Glu Arg Ile Gln Ala Glu Gln Val Asp
                405                 410                 415

Ala Val Thr Leu Ser Gly Glu Asp Ile Tyr Thr Ala Gly Lys Thr Tyr
            420                 425                 430

Gly Leu Val Pro Ala Ala Gly Glu His Tyr Ala Pro Glu Asp Ser Ser
        435                 440                 445

Asn Ser Tyr Tyr Val Val Ala Val Val Arg Arg Asp Ser Ser His Ala
    450                 455                 460

Phe Thr Leu Asp Glu Leu Arg Gly Lys Arg Ser Cys His Ala Gly Phe
465                 470                 475                 480

Gly Ser Pro Ala Gly Trp Asp Val Pro Val Gly Ala Leu Ile Gln Arg
                485                 490                 495

Gly Phe Ile Arg Pro Lys Asp Cys Asp Val Leu Thr Ala Val Ser Glu
            500                 505                 510

Phe Phe Asn Ala Ser Cys Val Pro Val Asn Asn Pro Lys Asn Tyr Pro
        515                 520                 525

Ser Ser Leu Cys Ala Leu Cys Val Gly Asp Glu Gln Gly Arg Asn Lys
    530                 535                 540

Cys Val Gly Asn Ser Gln Glu Arg Tyr Tyr Gly Tyr Arg Gly Ala Phe
545                 550                 555                 560

Arg Cys Leu Val Glu Asn Ala Gly Asp Val Ala Phe Val Arg His Thr
                565                 570                 575

Thr Val Phe Asp Asn Thr Asn Gly His Asn Ser Glu Pro Trp Ala Ala
            580                 585                 590

Glu Leu Arg Ser Glu Asp Tyr Glu Leu Leu Cys Pro Asn Gly Ala Arg
        595                 600                 605

Ala Glu Val Ser Gln Phe Ala Ala Cys Asn Leu Ala Gln Ile Pro Pro
    610                 615                 620

His Ala Val Met Val Arg Pro Asp Thr Asn Ile Phe Thr Val Tyr Gly
625                 630                 635                 640

Leu Leu Asp Lys Ala Gln Asp Leu Phe Gly Asp Asp His Asn Lys Asn
                645                 650                 655

Gly Phe Lys Met Phe Asp Ser Ser Asn Tyr His Gly Gln Asp Leu Leu
            660                 665                 670

Phe Lys Asp Ala Thr Val Arg Ala Val Pro Val Gly Glu Lys Thr Thr
        675                 680                 685

Tyr Arg Gly Trp Leu Gly Leu Asp Tyr Val Ala Ala Leu Glu Gly Met
    690                 695                 700

Ser Ser Gln Gln Cys Ser Gly Ala Ala Ala Pro Ala Pro Gly Ala Pro
705                 710                 715                 720

Leu Leu Pro Leu Leu Leu Pro Ala Leu Ala Ala Arg Leu Leu Pro Pro
                725                 730                 735

Ala Leu